(19)

Europäisches
Patentamt
European
Patent Office
Office européen
des brevets

(11)  **EP 2 431 032 A1**

(12)  # EUROPEAN PATENT APPLICATION
published in accordance with Art. 153(4) EPC

(43) Date of publication:
**21.03.2012  Bulletin 2012/12**

(21) Application number: **10774719.8**

(22) Date of filing: **11.05.2010**

(51) Int Cl.:
*A61K 31/282* (2006.01)    *A61K 31/404* (2006.01)
*A61K 31/4412* (2006.01)   *A61K 31/513* (2006.01)
*A61K 31/53* (2006.01)     *A61P 35/00* (2006.01)

(86) International application number:
**PCT/JP2010/003202**

(87) International publication number:
**WO 2010/131460 (18.11.2010 Gazette 2010/46)**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **12.05.2009  JP 2009115847**

(71) Applicant: **Taiho Pharmaceutical Co., Ltd.
Chiyoda-ku, Tokyo 101-8444 (JP)**

(72) Inventor: **FUKUSHIMA, Masakazu
Tokushima-shi
Tokushima 771-0194 (JP)**

(74) Representative: **Blodig, Wolfgang
Wächtershäuser & Hartz
Patentanwaltspartnerschaft
Weinstraße 8
80333 München (DE)**

(54)  **ANTI-TUMOR AGENT COMPRISING TEGAFUR-GIMERACIL-OTERACIL POTASSIUM
COMBINATION DRUG AND OXALIPLATIN**

(57)    To provide a novel combination therapy which exhibits remarkable anti-tumor effect.

The invention provides an anti-tumor agent comprising a combination drug containing tegafur, gimeracil and oteracil potassium, oxaliplatin, and TSU-68 or a salt thereof, in combination.

**EP 2 431 032 A1**

**Description**

Field of the Invention

The present invention relates to a novel anti-tumor agent which contains a combination drug containing tegafur, gimeracil and oteracil potassium, oxaliplatin (hereinafter may be also referred to as "1-OHP"), and 3-[2,4-dimethyl-5-[[(Z)-2,3-dihydro-2-oxo-1H-indol-3-ylidene]methyl]-1H-pyrrol-3-yl]propanoic acid (hereinafter may be also referred to as "TSU-68") or a salt thereof, and to a novel anti-tumor-effect-potentiator.

Background of the Invention

Conventionally, 5-fluorouracil (hereinafter abbreviated to "5-FU") has been basically employed for chemotherapy for advanced or recurrent colorectal cancer, and a so-called FOLFOX therapy which consists of 5-FU/leucovorin and 1-OHP is employed as a standard therapy (Non-Patent Document 1).

The efficacy and adverse side effect of 5-FU considerably varies depending on the administration method. Thus, prodrugs of 5-FU have been developed as cancer therapeutic drugs exhibiting greater efficacy or higher safety. Among such 5-FU prodrugs, a combination drug containing tegafur as a 5-FU prodrug (TS-1, product name (also called "S-1"), tegafur : gimeracil : oteracil potassium = 1 : 0.4 : 1 (by mole)) is widely used in clinical settings (Non-Patent Document 2). Furthermore, a variety of combination therapies have been developed for potentiating anti-tumor effect according to S-1. A study has revealed that a combination therapy with L-OHP exhibits a high therapeutic effect (Non-Patent Document 3).

As described above, the 5-FU/leucovorin therapy or S-1/1-OHP therapy exhibits high therapeutic effect on advanced or recurrent colorectal cancer and serves as a useful therapeutic method. However, in order to realize longer survival time of patients, a colorectal cancer therapy which exhibits more potent anti-tumor effect and give less adverse side effects is needed.

Meanwhile, TSU-68 is a drug which has been developed as a tyrosine kinase inhibitor against a receptor for an angiogenesis-related factor such as vascular endothelial growth factor (VEGF) or platelet-derived growth factor (PDGF). In in vivo trials of nude mice into which human cancer cells have been subcutaneously transplanted, TSU-68 exhibits a tumor growth suppressing effect on cancers such as lung cancer, colorectal cancer, and uterine cancer (Non-Patent Document 4). Combination use of S-1 and TSU-68 is known to potentiate the anti-tumor effect (Non-Patent Document 5). However, it has never been known that combination use of S-1, 1-OHP, and TSU-68 potentiates specifically the anti-tumor effect without potentiating adverse side effects.

Prior Art Documents

Non-Patent Documents

Non-Patent Document 1: J. Clin. Oncol. (2000); 18: 2938-47
Non-Patent Document 2: Cancer (2004); 100: 2355-61
Non-Patent Document 3: Br. J. Cancer (2008); 98(6): 1034-8
Non-Patent Document 4: Cancer Res. (2000); 60(15): 4152-60
Non-Patent Document 5: 60th Annual Meeting of the Japanese Cancer Association (2001); p. 580, No. 2024

Summary of the Invention

Problems to be Solved by the Invention

An object of the present invention is to provide a novel anti-tumor agent and an anti-tumor therapy which exhibit remarkable anti-tumor effect, particularly an effect of suppressing tumor growth.

Means for Solving the problems

In view of the foregoing, according to the present inventor's extensive studies on a novel combination therapy of an S-1/1-OHP combination therapy (hereinafter referred to as "S-1/1-OHP combination therapy") with another anti-tumor agent, in order to develop a cancer therapy which can elongate the survival time of patients, the inventor has found that when the S-1/1-OHP combination therapy is used in combination with TSU-68, which is a tyrosine kinase inhibitor to a receptor of an angiogenesis-related factor such as VEGF and PDGF, the anti-tumor effect of the S-1/1-OHP combination therapy is remarkably potentiated, and the onset of adverse side effects is not promoted. The present invention has been accomplished on the basis of this finding. In general, when one anti-tumor agent is used in combination with another anti-tumor agent, the anti-tumor effect is potentiated, and adverse side effects are also potentiated. Therefore, quite surprisingly, the present invention can realize both remarkably high anti-tumor effect, particularly an effect of retarding tumor growth, and reduced risks for onset of adverse side effects.

Accordingly, the present invention is directed to the following (1) to (14):

(1) An anti-tumor agent comprising, in combination, a combination drug containing tegafur, gimeracil and oteracil potassium, oxaliplatin, and 3-[2,4-dimethyl-5-[[(Z)-2,3-dihydro-2-oxo-1H-indol-3-ylidene]methyl]-1H-pyrrol-3-yl]propanoic acid or a salt thereof;

(2) The anti-tumor agent as described in (1) above, comprising tegafur, gimeracil and oteracil potassium at a molar ratio of 1 : 0.4 : 1;

(3) The anti-tumor agent as described in (2) above, comprising 60 to 120 mg/m$^2$ of the combination drug containing tegafur, gimeracil and oteracil potassium in terms of tegafur as a daily dose; 80 to 150 mg/m$^2$ of oxaliplatin as a daily dose; and 200 to 1,500 mg of 3-[2,4-dimethyl-5-[[(Z)-2,3-dihydro-2-oxo-1H-indol-3-ylidene]methyl]-1H-pyrrol-3-yl]propanoic acid or a salt thereof in terms of to 3-[2,4-dimethyl-5-[[(Z)-2,3-dihydro-2-oxo-1H-indol-3-ylidene]methyl]-1H-pyrrol-3-yl]propanoic acid as a daily dose;

(4) The anti-tumor agent as described in (3) above, comprising 70 mg/m$^2$ of the combination drug containing tegafur, gimeracil and oteracil potassium in terms of tegafur as a daily dose; 130 mg/m$^2$ of oxaliplatin as a daily dose; and 400 to 800 mg of 3-[2,4-dimethyl-5-[[(Z)-2,3-dihydro-2-oxo-1H-indol-3-ylidene]methyl]-1H-pyrrol-3-yl]propanoic acid or a salt thereof in terms of 3-[2,4-dimethyl-5-[[(Z)-2,3-dihydro-2-oxo-1H-indol-3-ylidene]methyl]-1H-pyrrol-3-yl]propanoic acid as a daily dose;

(5) The anti-tumor agent as described in any of (1) to (4) above, wherein each of oxaliplatin and 3-[2,4-dimethyl-5-[[(Z)-2,3-dihydro-2-oxo-1H-indol-3-ylidene]methyl]-1H-pyrrol-3-yl]propanoic acid or a salt thereof is in the form of a single drug;

(6) The anti-tumor agent as described in any of (1) to (4) above, which is a kit-type preparation comprising a combination drug containing tegafur, gimeracil and oteracil potassium, a preparation containing oxaliplatin, and a preparation containing 3-[2,4-dimethyl-5-[[(Z)-2,3-dihydro-2-oxo-1H-indol-3-ylidene]methyl]-1H-pyrrol-3-yl]propanoic acid or a salt thereof;

(7) An anti-tumor effect potentiator comprising 3-[2,4-dimethyl-5-[[(Z)-2,3-dihydro-2-oxo-1H-indol-3-ylidene]methyl]-1H-pyrrol-3-yl]propanoic acid or a salt thereof, for use in combination therapy employing a combination drug containing tegafur, gimeracil and oteracil potassium, and oxaliplatin;

(8) The anti-tumor effect potentiator as described in (7) above, comprising 200 to 400 mg of 3-[2,4-dimethyl-5-[[(Z)-2,3-dihydro-2-oxo-1H-indol-3-ylidene]methyl]-1H-pyrrol-3-yl]propanoic acid or a salt thereof in terms of 3-[2,4-dimethyl-5-[[(Z)-2,3-dihydro-2-oxo-1H-indol-3-ylidene]methyl]-1H-pyrrol-3-yl]propanoic acid per administration unit;

(9) Use, in combination, of a combination drug containing tegafur, gimeracil and oteracil potassium, oxaliplatin, and 3-[2,4-dimethyl-5-[[(Z)-2,3-dihydro-2-oxo-1H-indol-3-ylidene]methyl]-1H-pyrrol-3-yl]propanoic acid or a salt thereof, for the production of an anti-tumor agent;

(10) A combination of a combination drug containing tegafur, gimeracil and oteracil potassium, oxaliplatin, and 3-[2,4-dimethyl-5-[[(Z)-2,3-dihydro-2-oxo-1H-indol-3-ylidene]methyl]-1H-pyrrol-3-yl]propanoic acid or a salt thereof, for the treatment of cancer.

(11) A method for treatment of cancer, comprising administering, to a patient in need thereof, a combination drug containing tegafur, gimeracil and oteracil potassium, oxaliplatin, and 3-[2,4-dimethyl-5-[[(Z)-2,3-dihydro-2-oxo-1H-indol-3-ylidenelmethyll-1H-pyrro1-3-yllpropanoic acid or a salt thereof, in combination;

(12) The method as described in (11) above, wherein 60 to 120 mg/m$^2$ of the combination drug containing tegafur, gimeracil and oteracil potassium in terms of tegafur as a daily dose, 80 to 150 mg/m$^2$ of oxaliplatin as a daily dose, and 200 to 1,500 mg of 3-[2,4-dimethyl-5-[[(Z)-2,3-dihydro-2-oxo-1H-indol-3-ylidene]methyl]-1H-pyrrol-3-yl]propanoic acid or a salt thereof in terms of 3-[2,4-dimethyl-5-[[(Z)-2,3-dihydro-2-oxo-1H-indol-3-ylidene]methyl]-1H-pyrrol-3-yl]propanoic acid as a daily dose;

(13) A method for potentiating an anti-tumor effect in a combination therapy employing a combination drug containing tegafur, gimeracil and oteracil potassium and oxaliplatin, in combination, wherein the method comprises administering, to a patient in need thereof, 3-[2,4-dimethyl-5-[[(Z)-2,3-dihydro-2-oxo-1H-indol-3-ylidene]methyl]-1H-pyrrol-3-yl]propanoic acid or a salt thereof; and

(14) 3-[2,4-Dimethyl-5-[[(Z)-2,3-dihydro-2-oxo-1H-indol-3-ylidene]methyl]-1H-pyrrol-3-yl]propanoic acid or a salt thereof for use in potentiating an anti-tumor effect in a combination therapy employing a combination drug containing tegafur, gimeracil and oteracil potassium and oxaliplatin, in combination.

Effects of the Invention

The anti-tumor agent which comprises, in combination, a combination drug containing tegafur, gimeracil and oteracil potassium, 1-OHP, and TSU-68 or a salt thereof exhibits remarkably high anti-tumor effects such as reduction in tumor volume and suppression of tumor growth, as compared with conventional combination anti-tumor agents. Thus, through use of the anti-tumor agent of the present invention, lifetime can be elongated.

Brief Description of the Drawings

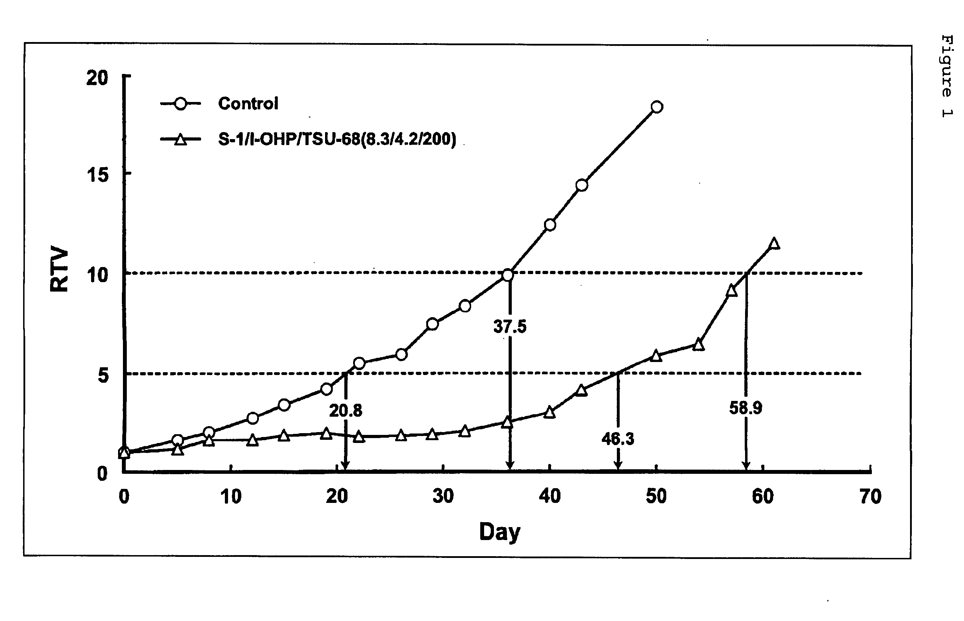

[Fig. 1] Effect of S-1/1-OHP/TSU-68 combination group on tumor growth delay in human colorectal cancer DLD-1 xenograft model.

Detailed Description of the Invention

As shown in the below Examples, use of a combination drug containing tegafur, gimeracil and oteracil potassium, in combination with 1-OHP and TSU-68 or a salt thereof, exhibits remarkably high anti-tumor effect while suppressing adverse side effects, as compared with using one ingredient or combination of two of the ingredients. Therefore, a combination of a combination drug containing tegafur, gimeracil and oteracil potassium, 1-OHP, and TSU-68 or a salt thereof can serve as an anti-tumor agent, and can be employed for the production of an anti-tumor agent, or can be used for cancer therapy wherein an effective amount of the combination is administered to a patient in need thereof. 3-[2,4-Dimethyl-5-[[(Z)-2,3-dihydro-2-oxo-1H-indol-3-ylidene]methyl]-1H-pyrrol-3-yl]propanoic acid or a salt thereof can serve as an anti-tumor effect potentiator in combination therapy employing a combination drug containing tegafur, gimeracil and oteracil potassium, and 1-OHP, and may be employed for potentiating an anti-tumor effect by administering an effective amount of the compound to a patient in need thereof.
In the present invention, the "anti-tumor effect" is evaluated as a tumor regression effect, a tumor-growth delay effect, or the like.
In the present invention, examples of the combination drug containing tegafur, gimeracil and oteracil potassium include a preparation produced by mixing tegafur, gimeracil and oteracil potassium at a specific ratio and optionally mixing the mixture with the below-mentioned pharmaceutically acceptable carrier.
"Tegafur" is a known compound, which is 5-fluoro-1-(2-tetrahydrofuryl)-2,4-(1H,3H)-pyrimidinedione. Tegafur serves as a prodrug which is activated in the living body and releases an active ingredient 5-FU. Tegafur may be produced through known methods, such as a method disclosed in JP-B-1974-10510.
"Gimeracil" is a known compound, which is 2,4-dihydroxy-5-chloropyridine. Gimeracil itself exhibits no anti-tumor activity, while suppresses inactivation of 5-FU via metabolism in the living body, in order to potentiate the anti-tumor effect.
"Oteracil potassium" is a known compound, which is monopotassium 1,2,3,4-tetrahydro-2,4-dioxo-1,3,5-triazine-6-carboxylate. Oteracil potassium itself exhibits no anti-tumor activity, while which is distributed to the digestive tract and suppresses activation of 5-FU in the digestive tract, in order to prevent digestive tract disorders.
The ratio of tegafur, gimeracil and oteracil potassium in the anti-tumor agent is not particularly limited, so long as the intended effects of individual ingredients can be maintained. For example, the content ratio of a known combination drug disclosed in Japanese Patent No. 2614164 may be employed. Specifically, gimeracil is used in an amount of about 0.1 to about 5 mol (preferably about 0.2 to about 1.5 mol), and oteracil potassium is used in an amount of about 0.1 to about 5 mol (preferably about 0.2 to about 2 mol), with respect to 1 mol of tegafur. More preferably, the ratio of the active ingredients, tegafur : gimeracil : oteracil potassium (by mole) is 1 : 0.4 : 1, which is a commercially available tegafur-gimeracil-oteracil potassium combination drug, namely "TS-1" (also called S-1).
"Oxaliplatin" (1-OHP) employed in the present invention is a known compound, which is cis-oxalato(1R,2R-diaminocyclohexane)platinum(II). 1-OHP binds to DNA in cancer cells, to thereby induce functional damage on DNA and cleavage of DNA strand, leading to death of the cancer cells. 1-OHP may be produced through a know method, for example, a method disclosed in JP-B-1985-41077.
The amount of 1-OHP incorporated into the anti-tumor agent of the present invention is not particularly limited, so long as the anti-tumor effect can be potentiated. For example, 1-OHP is used in an amount (daily amount) of about 0.1 to about 5.0 mol, preferably about 0.2 to about 3.0 mol, particularly preferably about 0.4 to about 2.0 mol, with respect to 1 mol of tegafur.
"3-[2,4-Dimethyl-5-[[(Z)-2,3-dihydro-2-oxo-1H-indol-3-ylidene]methyl]-1H-pyrrol-3-yl]propanoic acid" (TSU-68) employed in the present invention is a known VEGF and PDGF receptor-associated tyrosine kinase inhibitor and is known to exhibit an anti-tumor effect on solid cancers such as lung cancer, colorectal cancer, and uterus cancer.

TSU-68 or a salt thereof may be produced through a known method, such as a method disclosed in Japanese Patent No. 3231044.

The salt of TSU-68 is not particularly limited, so long as the salt is pharmaceutically acceptable. Examples of the salt include salts of inorganic acid such as hydrochloric acid salt, hydrobromic aid salt, sulfuric acid salt, nitric acid salt, or phosphoric acid salt; and salts of organic acid such as methanesulfonic acid salt, ethanesulfonic acid salt, p-toluenesulfonic acid salt, or salicylic acid salt.

The amount of TSU-68 or a salt thereof incorporated into the anti-tumor agent of the present invention is not particularly limited, so long as the anti-tumor effect can be potentiated. For example, TSU-68 or a salt thereof is used in an amount (daily amount) of about 0.1 to about 100 mol, preferably about 0.5 to about 50 mol, particularly preferably about 1 to about 10 mol, with respect to 1 mol of tegafur.

The anti-tumor agent of the present invention may be an admixture (combination drug) in which the combination drug containing tegafur, gimeracil and oteracil potassium, 1-OHP, and TSU-68 or a salt thereof (a plurality of active ingredients) are mixed at aforementioned ratio to form a single agent (one part type), or may be a multiple-part preparation containing respective active ingredients in the form of single agents (each containing a single active ingredient) or a combined agent (multiple part type), for enabling the ingredients to be administered simultaneously, or separately and intermittently. Among them, a preferred embodiment is a multiple part type drug comprising a single preparation containing 1-OHP, a single preparation containing TSU-68 or a salt thereof, and a separately prepared combination drug containing tegafur, gimeracil and oteracil potassium.

The cancer which can be effectively treated by use of the anti-tumor agent of the present invention is not particularly limited. Examples of the target cancer include colorectal cancer, liver cancer, kidney cancer, head and neck cancer, esophageal cancer, stomach cancer, biliary tract cancer, gallbladder/bile duct cancer, pancreatic cancer, lung cancer, breast cancer, ovarian cancer, cervical cancer, endometrial cancer, bladder cancer, prostatic cancer, testicular tumor, musculoskeletal sarcoma, leukemia, malignant lymphoma, multiple myeloma, skin cancer, and brain tumor. Among them, colorectal cancer, stomach cancer, head and neck cancer, lung cancer, breast cancer, pancreatic cancer, biliary tract cancer, and liver cancer are preferred, with colorectal cancer being particularly preferred.

The administration form of the preparation is not particularly limited, and may be appropriately selected depending on the purpose of the therapy. Specific examples of the administration form include oral agents (e.g., tablet, coated tablet, powder, granules, capsule, and liquid), injection, suppository, patch, and ointment. When the anti-tumor agent of the present invention is prepared as a multiple part type, these ingredients may be prepared as the same administration form or different administration forms. In one preferred embodiment, a preparation containing a tegafur-gimeracil-oteracil potassium combination drug and a preparation containing TSU-68 or a salt thereof may be prepared in the form of oral agent, and a preparation containing 1-OHP may be prepared in the form of an agent for injection.

The anti-tumor agent of the present invention may be produced, packed and provided as separate agents, so long as the tegafur-gimeracil-oteracil potassium combination drug, 1-OHP, and TSU-68 or a salt thereof are administered in combination, or all or a part of the preparations may be produced, packed, and provided as a single-package product (i.e., kit preparation) suitable for combined administration.

Each preparation containing the above active ingredient of the present invention may be produced through a known method using a pharmaceutically acceptable carrier. The pharmaceutically acceptable carrier may be selected from carries generally employed in drugs. Examples of the carrier include a vehicle, a binder, a disintegrant, a lubricant, a diluent, a solubilizer, a suspending agent, a tonicity agent, a pH-regulator, a buffer, a stabilizer, a colorant, a flavoring agent, and a corrigent.

Examples of the vehicle include lactose, sucrose, sodium chloride, glucose, maltose, mannitol, erythritol, xylitol, maltitol, inositol, dextran, sorbitol, albumin, urea, starch, calcium carbonate, kaolin, crystalline cellulose, silicic acid, methylcellulose, glycerin, sodium alinate, gum arabic, and mixtures thereof. Examples of the lubricant include refined talc, stearate salts, borax, polyethylene glycol, and mixtures thereof. Examples of the binder include simple syrup, liquid glucose, liquid starch, liquid gelatin, polyvinyl alcohol, polyvinyl ether, polyvinylpyrrolidone, carboxymethyl cellulose, shellac, methyl cellulose, ethyl cellulose, water, ethanol, potassium phosphate, and mixtures thereof. Examples of the disintegrant include dry starch, sodium alginate, agar powder, laminaran powder, sodium hydrogencarbonate, calcium carbonate, polyoxyethylene sorbitan fatty acid esters, sodium lauryl sulfate, monoglyceryl stearate, starch, lactose, and mixtures thereof. Examples of the diluent include water, ethyl alcohol, macrogol, propylene glycol, ethoxylated isostearyl alcohol, polyoxy isostearyl alcohol, polyoxyethylene sorbitan fatty acid esters, and mixtures thereof. Examples of the stabilizer include sodium pyrosulfite, EDTA, thioglycolic acid, thiolactic acid, and mixtures thereof. Examples of the tonicity agent include sodium chloride, boric acid, glucose, glycerin, and mixtures thereof. Examples of the pH-regulator or buffer include sodium citrate, citric acid, sodium acetate, sodium phosphate, and mixtures thereof. Examples of the anesthetic agent include procaine hydrochloride lidocaine hydrochloride, and mixtures thereof.

The dose amount of the above active ingredient of the anti-tumor agent of the present invention is not particularly limited, so long as the intended effect of the each ingredient can be obtained. The dose amount is appropriately determined according to the agent of the patient, type of cancer, phase, the presence of metastasis, therapy history, administration of another anti-tumor agent, etc. In one embodiment, the dose amount of tegafur is 20 to 500 mg/m$^2$ (body surface area) /day, preferably 60 to 120 mg/m$^2$/day, particularly preferably 70 mg/m$^2$/day. The dose amount of gimeracil is 5.8 to 145 mg/m$^2$/day, preferably 17.4 to 34.8 mg/m$^2$/day, particularly preferably 20.3 mg/m$^2$/day. The dose amount of oteracil potassium is 19.6 to 490 mg/m$^2$/day, preferably 58.8 to 117.6 mg/m$^2$/day, particularly preferably 68.6 mg/m$^2$/day. The dose amount of 1-OHP is 40 to 300 mg/m$^2$/day, preferably 80 to 150 mg/m$^2$/day, particularly preferably 130 mg/m$^2$/day. The dose amount of TSU-68 or a salt thereof (as reduced to TSU-68) is 50 to 3000 mg/day, preferably 200 to 1,500 mg/day, particularly preferably 400 to 800 mg/day.

Thus, the anti-tumor agent of the present invention preferably may be prepared such that the combination drug containing tegafur, gimeracil and oteracil potassium, 1-OHP, and TSU-68 or a salt thereof are administered at the aforementioned daily doses.

In other words, the combination drug containing tegafur, gimeracil and oteracil potassium is administered at a daily dose (in terms of tegafur) of 60 to 120 mg/m$^2$, preferably 70 mg/m$^2$. 1-OHP is administered at a daily dose of 80 to 150 mg/m$^2$, preferably 130 mg/m$^2$. TSU-68 or a salt thereof is administered at a daily dose (in terms of TSU-68 or a salt thereof) of 200 to 1,500 mg, preferably 400 to 800 mg.

The regimen (order of administration and intervals thereof) of each active ingredient of the present invention is not particularly limited, so long as intended synergistic effect can be obtained. As an example of administration schedule (1 cycle = 21 days), S-1 is administered from Day 1 to Day 14, 1-OHP is administered on Day 1, and TSU-68 or a salt thereof is administered from Day 1 to Day 21, and the cycle is performed once or repeated plural times. When the anti-tumor agent of the present invention is employed for production of a kit-type preparation, individual preparations (active ingredients) may be administered simultaneously or separately.

TSU-68 or a salt thereof employed in the present invention exhibits an anti-tumor effect when used alone. However, through use of TSU-68 in combination with S-1/1-OHP, the anti-tumor effect attained by the S-1/1-OHP combination therapy can be more remarkably potentiated. Therefore, TSU-68 or a salt thereof serves as a useful anti-tumor effect potentiator in the S-1/1-OHP combination therapy. From the viewpoints of obtaining the anti-tumor effect potentiating effect in the S-1/1-OHP combination therapy and allowance of adverse side effects, the TSU-68 or a salt thereof (in terms of TSU-68) per unit dose is preferably 200 mg to 400 mg (400 mg to 800 mg as a daily dose).

Examples

The present invention is exemplified in detail by way of example, which should not be construed as limiting the invention thereto.

Example 1: Combination effect of TSU-68 on anti-tumor effect of S-1 + 1-OHP in human colorectal cancer COL-1 xenograft model.

A fragment (about 2 mm diameter) of human colorectal cancer COL-1 was transplanted into the right axilla of each of male nude mice (BALB/cA-nu, 4-week old, purchased from CLEA Japan, Inc.), and the tumor volume ((longer diameter)x(shorter diameter)$^2$/2) of the mouse was calculated. The mice were allocatedinto groups so that the average tumor volumes of the groups were adjusted to about 220 mm$^3$ (8 mice/group). Drug administration was started on the next day after the day of grouping (Day 0) .

S-1 was orally administered once a day at a dose (as in terms of tegafur) of 7.0 mg/kg/day from Day 1 to Day 28. 1-OHP was administered, once a week, to each mouse through the tail vein at a dose of 4.2 mg/kg/day from Day 1 to Day 28. TSU-68 was orally administered once a day at a dose of 200 or 400 mg/kg/day from Day 1 to Day 28. A group to which no drug was administered was set as a control group.

During drug administration, the tumor volume of each mouse was measured every 3 to 4 days, and the relative tumor volume (RTV) and the anti-tumor effect (IR (%)) of each group were calculated by the formulas below. The result is shown in Table 1. The statistical significance of difference of the anti-tumor effect between drug-administration group and control group was analyzed by the Student's t-test (both sides) using the average RTV value of these groups. A P value of $p<0.05$ was considered statistically significant. Furthermore, the advantage in anti-tumor effect (augmentation effect) of the S-1/1-OHP/TSU-68 combination group, with respect to each of the control group, the S-1/1-OHP combination group and the TSU-68 single administration group, was statistically analyzed through the IUT test. The effect of combination use was confirmed when the maximum of the significance level of any of the compared groups (overall maximal p value) was lower than 0.05 and the average RTV value of the 3-drug combination group was lower than that of the S-1/1-OHP combination group or a single drug administration group.

[F1]

$$\text{Relative tumor volume (RTV)} = \frac{\text{(Tumor volume on Day N)}}{\text{(Tumor volume on Day 1)}}$$

$$\text{Anti-tumor effect (IR(\%))} = [1 - \frac{\text{(RTV of drug-administration group)}}{\text{(RTV of control group)}}] \times 100$$

[Table 1]

Anti-tumor effect of S-I/I-OHP/TSU-68 combination group in Xenograft model of COL-1 human colorectal cancer in

| Drug | Dose (mg/kg) | | | N | IR (%) | | |
|------|------|------|------|---|--------|--------|--------|
| | S-1 | OHP | TSU | | Day 15 | Day 22 | Day 29 |
| S-1/OHP | 7 | 4.2 | - | 8 | 43.5 | 43.1 | 54.7 |
| TSU-68 | - | - | 200 | 8 | 47.5 | 47.8 | 49.2 |
| | - | - | 400 | 8 | 51.4 | 46.3 | 52.8 |
| S-1/OHP/TSU | 7 | 4.2 | 200 | 8 | 58.7[1] | 54.4[1] | 64.4[1] |
| | 7 | 4.2 | 400 | 8 | 66.6[1] | 64.1[1] | 69.5[1] |

1): Overall maximal $P<0.05$ (*IUT test*)

The IR values of the S-1/1-OHP/TSU-68 combination groups (200 mg group and 400 mg group) on Day 15, Day 22, and Day 29 were statistically significantly higher than those of the S-1/1-OHP combination group and the TSU-68 groups (200 mg group and 400 mg group). Thus, significant potentiation of the anti-tumor effect was statistically confirmed when S-1/1-OHP was administered in combination with TSU-68.

In addition to tumor volume, the body weight of each mouse was measured, and the percent body weight change (BWC (%)) of each group was calculated by the below formula.

[F2]

$$\text{Percent body weight change (BWC (\%))} = [\frac{\text{((Body weight on Day N)} - \text{(Body weight on Day 0))}}{\text{(Body weight on Day 0)}}] \times 100$$

As a result, the percent body weight change values of the S-1/1-OHP/TSU-68 combination groups (200 mg group and 400 mg group) on Day 22 and Day 29 were -2.50% and -1.86% (Day 22) and -2.31% and -1.26% (Day 29), as compared with the control group, and +1.38% and +2.02% (Day 22) and -0.8% and +0.2% (Day 29), as compared with the S-1/1-OHP combination group. Thus, in the present invention, body weight loss was confirmed to be equal to or less than that of each comparative case, and administration of S-1/1-OHP in combination with TSU-68 was confirmed to prevent augmentation of adverse side effects (e.g., body weight loss) at minimum.

Example 2: Effect of the combination of S-1 + 1-OHP + TSU-68 in human colorectal cancer DLD-1 Xenograft model

A fragment (about 2 mm diameter) of human colorectal cancer DLD-1 was transplanted to the right axilla of each of male nude mice (BALB/cA-nu, 4-week old, purchased from CLEA Japan, Inc.), and the tumor volume ((longer diameter)×(shorter diameter)$^2$/2) of the mouse was calculated. The mice were divided into groups so that the average tumor volumes of the groups were adjusted to about 220 mm$^3$ (8 mice/group). Drug administration was started on the next day after the day of grouping (Day 0).

S-1 was orally administered once a day at a dose (as in terms of tegafur) of 8.3 mg/kg/day from Day 1 to Day 21. 1-OHP was administered, once a week, to each mouse through the tail vein at a dose of 4.2 mg/kg/day from Day 1 to Day 21. TSU-68 was orally administered once a day at a dose of 200 mg/kg/day from Day 1 to Day 21. A group to which no drug was administered was set as a control group.

During drug administration, the tumor volume of each mouse was measured every 3 to 4 days, and the relative tumor volume (RTV) of each group was calculated by the formula below. The result is shown in a graph of Fig. 1, in which the horizontal axis represents administration period (days) and the vertical axis represents relative tumor volume. The statistical significance of difference of the anti-tumor effect between drug administration group and control group was analyzed by the Student's t-test (both sides) using the average RTV value of these groups. A P value of <0.05 was considered statistically significant. In Example 2, the period of time (days) when the tumor volume of each group (control group or S-1 + 1-OHP + TSU-68 combination group) is reached to 5 times or 10 times larger than that of at the start of the treatment was measured, in order to evaluate the tumor growth delay effect.

As is clear from the graph, the S-1 + 1-OHP + TSU-68 combination group exhibited a significant anti-tumor effect as compared with the control group, without causing severe adverse side effects (Anti-tumor effect (IR) of the TSU-68 combination group at the end of the therapy: 67.2%).

The period of time (days) when the tumor size (volume) reached to 5 times and 10 times the tumor volume at the start of the treatment was measured. In the case of the control group, 20.8 days and 37.5 days were required, respectively, and in the case of S-1 + 1-OHP + TSU-68 combination group, 46.3 days and 58.9 days were required, respectively. Thus, the tumor growth period was found to be delayed by combination administration.

[F3]

$$\text{Relative tumor volume (RTV)} = (\text{Tumor volume on Day N})/(\text{Tumor volume on Day 1})$$

$$\text{Anti-tumor effect (IR(\%))} = [1 - (\text{RTV of drug-administration group})/(\text{RTV of control group})] \times 100$$

As described above, the S-1/1-OHP/TSU-68 combination group was confirmed to serve as a cancer therapy which provides remarkably potentiated anti-tumor effect with suppressing adverse side effects and which can suppress tumor growth over a long period of time.

Example 3: Recommended dose of TSU-68 for metastatic colorectal cancer patients in S-1/1-OHP/TSU-68 combination therapy

The safety of the case where metastatic colorectal cancer patients (9 cases) were subjected to 1-OHP and S-1 combination therapy with administration of TSU-68 was examined. In this study, one cycle of the treatment was consisted of 21 days. To each patient, 1-OHP was intravenously administered at a dose of 130 mg/m$^2$ (per body surface area) on Day 1, and S-1 was orally administered, twice a day after meal, at a daily dose (in terms of tegafur) of 70 mg/m$^2$ (per body surface area) from Day 1 to Day 14.

The 7 days from Day 15 to Day 21 were placed as a rest period. TSU-68 was orally administered every day, twice a day after meal, at a daily dose of 400 mg to 800 mg.

The above study was carried out in order to determine the recommended dose (RD) of TSU-68 in the case where 1-OHP and S-1 combination therapy, whose efficacy to colorectal cancer had been established, may be carried out in combination with administration of TSU-68, without causing adverse side effects.

The safety was evaluated according to NCI-CTCAE (3rd edition, the National Cancer Institute's Common Terminology Criteria for Adverse Events version 3.0).

In the TSU-68 800 mg group, dose-limiting toxicity (DLT) including singultus and skin response in the hands and feet (Grade 3) or neutropenia (Grade 2) was observed, whereas no DLT was observed in the 400 mg group. The anti-tumor effect was almost equivalent between the 400 mg group and the 800 mg group. Therefore, the RD of TSU-68 in the combination therapy was determined to be 400 mg. Thus, additional administration of TSU-68 to 1-OHP and S-1 combination therapy would be a useful combination therapy for colorectal cancer patients.

As described above, the test scores of the S-1/1-OHP/TSU-68 combination group indicated that the combination therapy is a cancer therapy exhibiting a remarkably increased anti-tumor effect while adverse side effects are suppressed.

**Claims**

1. An anti-tumor agent comprising a combination of a combination drug containing tegafur, gimeracil and oteracil potassium, oxaliplatin, and 3-[2,4-dimethyl-5-[[(Z)-2,3-dihydro-2-oxo-1H-indol-3-ylidene]methyl]-1H-pyrrol-3-yl]propanoic acid or a salt thereof.

2. The anti-tumor agent according to claim 1, comprising tegafur, gimeracil and oteracil potassium at a molar ratio of 1 : 0.4 : 1.

3. The anti-tumor agent according to claim 2, comprising 60 to 120 mg/m$^2$ of the combination drug containing tegafur, gimeracil and oteracil potassium in terms of tegafur as a daily dose; 80 to 150 mg/m$^2$ of oxaliplatin as a daily dose; and 200 to 1,500 mg of 3-[2,4-dimethyl-5-[[(Z)-2,3-dihydro-2-oxo-1H-indol-3-ylidene]methyl]-1H-pyrrol-3-yl]propanoic acid or a salt thereof in terms of 3-[2,4-dimethyl-5-[[(Z)-2,3-dihydro-2-oxo-1H-indol-3-ylidene]methyl]-1H-pyrrol-3-yl]propanoic acid as a daily dose.

4. The anti-tumor agent according to claim 3, comprising 70 mg/m$^2$ of the combination drug containing tegafur, gimeracil and oteracil potassium in terms of tegafur as a daily dose; 130 mg/m$^2$ of oxaliplatin as a daily dose; and 400 to 800 mg of 3-[2,4-dimethyl-5-[[(Z)-2,3-dihydro-2-oxo-1H-indol-3-ylidene]methyl]-1H-pyrrol-3-yl]propanoic acid or a salt thereof in terms of 3-[2,4-dimethyl-5-[[(Z)-2,3-dihydro-2-oxo-1H-indol-3-ylidene]methyl]-1H-pyrrol-3-yl]propanoic acid as a daily dose.

5. The anti-tumor agent according to any one of claims 1 to 4, wherein each of oxaliplatin and 3-[2,4-dimethyl-5-[[(Z)-2,3-dihydro-2-oxo-1H-indol-3-ylidene]methyl]-1H-pyrrol-3-yl]propanoic acid or a salt thereof is in the form of a single drug.

6. The anti-tumor agent according to any one of claims 1 to 4, which is a kit-type preparation comprising a combination drug containing tegafur, gimeracil and oteracil potassium, a preparation containing oxaliplatin, and a preparation containing 3-[2,4-dimethyl-5-[[(Z)-2,3-dihydro-2-oxo-1H-indol-3-ylidene]methyl]-1H-pyrrol-3-yl]propanoic acid or a salt thereof.

7. An anti-tumor effect potentiator comprising 3-[2,4-dimethyl-5-[[(Z)-2,3-dihydro-2-oxo-1H-indol-3-ylidene]methyl]-1H-pyrrol-3-yl]propanoic acid or a salt thereof, for use in combination therapy employing a combination drug containing tegafur, gimeracil and oteracil potassium, and oxaliplatin.

8. The anti-tumor effect potentiator according to claim 7, comprising 200 to 400 mg of 3-[2,4-dimethyl-5-[[(Z)-2,3-dihydro-2-oxo-1H-indol-3-ylidene]methyl]-1H-pyrrol-3-yl]propanoic acid or a salt thereof in terms of 3-[2,4-dimethyl-5-[[(Z)-2,3-dihydro-2-oxo-1H-indol-3-ylidene]methyl]-1H-pyrrol-3-yl]propanoic acid per administration unit.

9. Use, in combination, of a combination drug containing tegafur, gimeracil and oteracil potassium, oxaliplatin, and 3-[2,4-dimethyl-5-[[(Z)-2,3-dihydro-2-oxo-1H-indol-3-ylidenelmethyll-1H-pyrro1-3-yllpropanoic acid or a salt thereof, for the production of an anti-tumor agent.

10. A combination of a combination drug containing tegafur, gimeracil and oteracil potassium, oxaliplatin, and 3-[2,4-dimethyl-5-[[(Z)-2,3-dihydro-2-oxo-1H-indol-3-ylidene]methyl]-1H-pyrrol-3-yl]propanoic acid or a salt thereof, for the treatment of cancer.

11. A method for treatment of cancer, comprising administering, to a patient in need thereof, a combination drug containing tegafur, gimeracil and oteracil potassium, oxaliplatin, and 3-[2,4-dimethyl-5-[[(Z)-2,3-dihydro-2-oxo-1H-indol-3-ylidene]methyl]-1H-pyrrol-3-yl]propanoic acid or a salt thereof, in combination.

12. The method according to claim 11, wherein 60 to 120 mg/m$^2$ of the combination drug containing tegafur, gimeracil, and oteracil potassium in terms of tegafur as a daily dose, 80 to 150 mg/m$^2$ of oxaliplatin as a daily dose, and 200 to 1,500 mg of 3-[2,4-dimethyl-5-[[(Z)-2,3-dihydro-2-oxo-1H-indol-3-ylidene]methyl]-1H-pyrrol-3-yl]propanoic acid or a salt thereof in terms of 3-[2,4-dimethyl-5-[[(Z)-2,3-dihydro-2-oxo-1H-indol-3-ylidene]methyl]-1H-pyrrol-3-yl]propanoic acid as a daily dose.

13. A method for potentiating an anti-tumor effect in a combination therapy employing a combination drug containing tegafur, gimeracil and oteracil potassium and oxaliplatin, in combination, wherein the method comprises administering, to a patient in need thereof, 3-[2,4-dimethyl-5-[[(Z)-2,3-dihydro-2-oxo-1H-indol-3-ylidene]methyl]-1H-pyrrol-3-yl]propanoic acid or a salt thereof.

14. 3-[2,4-Dimethyl-5-[[(Z)-2,3-dihydro-2-oxo-1H-indol-3-ylidene]methyl]-1H-pyrrol-3-yl]propanoic acid or a salt thereof for use in potentiating an anti-tumor effect in a combination therapy employing a combination drug containing tegafur, gimeracil and oteracil potassium and oxaliplatin, in combination.

Figure 1

**INTERNATIONAL SEARCH REPORT**

| International application No. |
| --- |
| PCT/JP2010/003202 |

A. CLASSIFICATION OF SUBJECT MATTER
*A61K31/282*(2006.01)i, *A61K31/404*(2006.01)i, *A61K31/4412*(2006.01)i,
*A61K31/513*(2006.01)i, *A61K31/53*(2006.01)i, *A61P35/00*(2006.01)i

According to International Patent Classification (IPC) or to both national classification and IPC

B. FIELDS SEARCHED

Minimum documentation searched (classification system followed by classification symbols)
A61K31/282, A61K31/404, A61K31/4412, A61K31/513, A61K31/53, A61P35/00

Documentation searched other than minimum documentation to the extent that such documents are included in the fields searched
Jitsuyo Shinan Koho        1922-1996    Jitsuyo Shinan Toroku Koho    1996-2010
Kokai Jitsuyo Shinan Koho    1971-2010    Toroku Jitsuyo Shinan Koho    1994-2010

Electronic data base consulted during the international search (name of data base and, where practicable, search terms used)
CAPlus/REGISTRY/MEDLINE/EMBASE/BIOSIS(STN),
JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
| --- | --- | --- |
| Y | Hidenori FUJITA et al., "Shinki Keiko Kekkan Shinsei Sogaizai TSU-68(SU006668) no Kakushu Koganzai tono Heiyo Koka", Dai 60 Kai The Japanese Cancer Association Sokai Kiji, 25 August 2001 (25.08.2001), page 580 | 1-9,14 |
| Y | Manabu OTA et al., "Tyrosine Kinase Sogai Kekkan Shinsei Sogaizai TSU68 no Ko Shuyo Koka no Kento", Dai 109 Kai Annual Congress of Japan Surgical Society Shorokushu, 25 February 2009 (25.02.2009), page 649 | 1-9,14 |

| ☒ Further documents are listed in the continuation of Box C. | ☐ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 02 June, 2010 (02.06.10) | 29 June, 2010 (29.06.10) |

| Name and mailing address of the ISA/ | Authorized officer |
| --- | --- |
| Japanese Patent Office | |
| Facsimile No. | Telephone No. |

Form PCT/ISA/210 (second sheet) (July 2009)

**INTERNATIONAL SEARCH REPORT**

International application No.

PCT/JP2010/003202

C (Continuation). DOCUMENTS CONSIDERED TO BE RELEVANT

| Category* | Citation of document, with indication, where appropriate, of the relevant passages | Relevant to claim No. |
|---|---|---|
| Y | Yusuke DOI et al., "S-1 metronomic chemotherapy to Oxaliplatin Funyu Nano Carrier Heiyo Ryoho no Kaihatsu: Sojoteki Koka Zokyo Kiko no Kaimei ni Kansuru Kenkyu", Dai 46 Kai Japan Societyof Clinical Oncology Sokai Shorokugo, 01 October 2008 (01.10.2008), page 385 | 1-9,14 |
| Y | Li, J. et al, Phase I dose-escalating study of S-1 in combination with oxaliplatin for patients with advanced and/or metastatic colorectal cancer, Anti-Cancer Drugs, 2008, Vol.19, No.7, pp.745-748 | 1-9,14 |
| Y | Yamada, Y. et al, Phase I/II study of oxaliplatin with oral S-1 as first-line therapy for patients with metastatic colorectal cancer., British Journal of Cancer, 2008.05.25, Vol. 98, No. 6, pp. 1034-1038 | 1-9,14 |
| Y | Yuji INOUE et al., "Daicho Gan Kan Ten'i Kansetsusho Go Zankan Saihatsu ni Taisuru Chiryo", Shokaki Geka, 2006, vol.29, pages 1169 to 1174 | 1-9,14 |
| Y | Oh, S.Y. et al, Phase II Trial of S-1 in Combination with Oxaliplatin in Previously Untreated Patients with Recurrent or Inoperable Biliary Tract Cancer, Chemotherapy, 2008, Vol.54, pp.479-484 | 1-9,14 |
| Y | JP 2003-535038 A (Sugen, Inc.), 25 November 2003 (25.11.2003), entire text & US 2003/0191162 A1 & WO 2000/038519 A1 | 1-9,14 |
| A | Shizuo MACHIDA et al., "Ranso Gan Fukumaku Hashu ni Taisuru Kekkan Shinsei Sogaizai SU6668(TSU-68) Keiko Toyo no Kisoteki Kento", Japanese Journal of Cancer Clinics, 2005, vol.51, no.4, pages 279 to 284 | 1-9,14 |

Form PCT/ISA/210 (continuation of second sheet) (July 2009)

<div align="center">**INTERNATIONAL SEARCH REPORT**</div>

| International application No. |
| --- |
| PCT/JP2010/003202 |

**Box No. II      Observations where certain claims were found unsearchable (Continuation of item 2 of first sheet)**

This international search report has not been established in respect of certain claims under Article 17(2)(a) for the following reasons:

1. ☒ Claims Nos.: 10-13
   because they relate to subject matter not required to be searched by this Authority, namely:
   Claims 10 to 13 involve "methods for treatment of the human body or animal body by surgery or therapy".

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 49010510 B
- JP 2614164 B
- JP 60041077 B
- JP 3231044 B

**Non-patent literature cited in the description**

- *J. Clin. Oncol.,* 2000, vol. 18, 2938-47
- *Cancer,* 2004, vol. 100, 2355-61
- *Br. J. Cancer,* 2008, vol. 98 (6), 1034-8
- *Cancer Res.,* 2000, vol. 60 (15), 4152-60
- *60th Annual Meeting of the Japanese Cancer Association,* 2001, 580